# FASCICULE DE BREVET EUROPEEN

(11) **EP 1 663 462 B1**
(45) Date de publication et mention de la délivrance du brevet: **22.05.2013**
(21) Numéro de dépôt: 04786329.5
(22) Date de dépôt: 19.08.2004
(51) Int. Cl.: B01D 71/02, B01D 53/22, B01J 23/00, C01B 13/02

(54) **MEMBRANES DE CONDUCTION D OXYGENE, PROCEDE DE PREPARATION, AINSI QUE REACTEUR ET PROCEDE UTILISANT LES MEMBRANES**
SAUERSTOFFLEITMEMBRANE, HERSTELLUNGSVERFAHREN DAFÜR UND REAKTOR SOWIE VERWENDUNGSVERFAHREN DAFÜR
OXYGEN CONDUCTING MEMBRANES, PREPARATION METHOD THEREOF, AND REACTOR AND METHOD USING SAME

(30) Priorité: 28.08.2003 FR 0310258
(43) Date de publication de la demande: 07.06.2006
(73) Titulaire: CENTRE NATIONAL DE LA RECHERCHE SCIENTIFIQUE (CNRS), 75016 Paris (FR)
(72) Inventeur: FARRUSSENG, David, F-69300 CALUIRE (FR); MIRODATOS, Claude, F-69003 LYON (FR); REBEILLEAU, Michael, F-69007 LYON (FR); VAN VEEN, André, 69003 LYON (FR); Rushworth, Simon, Irby CH61 3XR (GB); ROUSSET, Jean-Luc, F-69006 LYON (FR)
(74) Mandataire: Colombet, Alain André
(86) Numéro de dépôt international: PCT/FR2004/002163
(87) Numéro de publication internationale: WO 2005/023402

(56) Documents cités:
- EP-A- 0 663 231
- EP-A- 0 766 330
- EP-A- 0 908 227
- DATABASE WPI Week 200344 Derwent Publications Ltd., London, GB; AN 2003-457993 XP002288952 & CN 1 403 196 A (HUANG Z) 19 mars 2003 (2003-03-19)
- HAIHUI WANG ET AL: CHEM. COMMUN., 2002, page 1468-1469, XP001166951 cité dans la demande
- Z.SHAO ET AL: ""investigation of the permeation behavior and stability of a Ba0.5Sr0.5Co0.8Fe0.0.2O3(delta) oxygen membrane"" JOUNAL OF MEMBRANE SCIENCE, vol. 172, no. 1-2, 2000, page 177-188, XP001166952 AMSTERDAM,NL cité dans la demande
- HIROMICHI SAKAMOTO ET AL.: SCIENCE AND TECHNOLOGY OF ADVANCED MATERIALS, 20 janvier 2003 (2003-01-20), pages 69-76, XP008041732 cité dans la demande
- J.L. ROUSSET ET AL.: PHYSICAL REVIEW B, vol. 58, no. 4, 15 juillet 1998 (1998-07-15), pages 2150-2156, XP008041798 cité dans la demande

## Description

L'invention concerne des membranes possédant des propriétés de conduction d'électrons et d'ions oxygène, leur procédé de préparation et leurs utilisations, notamment pour la déshydrogénation oxydante d'alcanes en alcènes.

Les procédés de déshydrogénation oxydante permettant la conversion des composés organiques saturés en composés insaturés sont bien connus. De nombreux efforts visant à améliorer de tels procédés ont été développés ces dernières années afin d'augmenter notamment les taux de conversion et la sélectivité vis-à-vis des produits désirés.

L'éthylène est produit industriellement par craquage thermique de l'éthane, généralement en présence de vapeur d'eau, lequel consiste en une pyrogénation de l'éthane à des températures élevées de l'ordre de 850°C. A l'heure actuelle, le taux de sélectivité en éthylène obtenu selon ce procédé n'excède pas 80 %, et le rendement en éthylène est de l'ordre de 60%. Bien qu'une augmentation de la température permette d'atteindre des taux de conversion supérieurs, cette élévation induit cependant une diminution de la sélectivité en favorisant la formation de produits secondaires et leur décomposition sous forme de coke. Or le dépôt de coke sur les parois des tuyaux de l'installation induit principalement deux effets néfastes : d'une part, il entraîne des chutes de pression à travers les tuyaux de l'installation et d'autre part il oppose une résistance au transfert de chaleur vers le fluide d'hydrocarbure.

La formation de coke constitue l'un des principaux facteurs limitant des opérations de craquage.

Pour tenter de pallier cette limitation, des procédés de déshydrogénation oxydante de l'éthane sont développés.

On distingue essentiellement deux types de procédé de déshydrogénation oxydante : les procédés catalytiques utilisant des lits de catalyseurs et ceux utilisant des membranes de conduction mixte.

Les principales réactions mises en jeu dans le procédé catalytique sont les suivantes :
C₂H₆ ↔ C₂H₄ + H₂
H₂ + ½ O₂ → H₂O
C₂H₆ + ⁷/₂O₂ → 2CO₂ + 3H₂O

Des catalyseurs permettant d'atteindre des rendements de transformation d'éthane en éthylène de l'ordre de 45-50 % à des températures notamment inférieures à 700°C ont récemment été décrits.

Ainsi, Thorsteinson et al. (J. Catal., 52, 1978, 116) ont obtenu un rendement en éthylène de 25 % avec une sélectivité de 90 % en utilisant le catalyseur MoVNbTeOx à 350°C.

Des rendements supérieurs compris entre 44 et 50% ont aussi été rapportés par Ji et Liu et al. (L. Ji, J. Liu, Chem. Commun., 1996, 1203) and Velle et al. (O.J. Velle, A. Andersen, K.-J. Jens, Catal. Today, 6 (1990) 567) avec les catalyseurs La/CaO, Li/La/CaO et SrCe_{0,5}Yb_{0,5}O_{2,75} dans un réacteur à lit fixe, mais à des températures de l'ordre de 600-700°C.

Cependant, pour obtenir un tel rendement, il est nécessaire d'opérer sous atmosphère enrichie en oxygène, ce qui induit une augmentation des coûts opérationnels.

De façon générale, s'ils permettent de résoudre le problème de formation de coke, les procédés catalytiques de déshydrogénation oxydante sont néanmoins difficilement exploitable industriellement à l'heure actuelle. Ils présentent en effet un certain nombre d'inconvénients, dont notamment des rendements inférieurs à ceux obtenus par les procédés commerciaux, la consommation de l'hydrogène produit, la formation de produits secondaires oxygénés, le caractère inflammable du mélange réactionnel, la nécessité d'enrichissement en oxygène et les coût qui en découlent.

Les procédés de déshydrogénation oxydante sélective d'éthane en éthylène utilisant des membranes denses de conduction d'ions oxygène se révèlent beaucoup plus prometteurs.

Des membranes denses de conduction sont des membranes capables de transporter sélectivement des ions oxygène à des températures généralement supérieures à 600°C, le plus souvent comprises entre 700°C et 1200°C.

Parmi les membranes denses de conduction d'oxygène, on distingue notamment les membranes composées d'électrolyte solide, de type ionique et les membranes de conduction mixte ionique-électronique à base d'oxydes multimétalliques.

Les membranes formées d'électrolyte solide sont composées d'oxydes inorganiques, typiquement d'oxydes de calcium ou de zirconium stabilisés par l'yttrium (YSZ), et possèdent généralement une structure perovskite ou fluorite.

Ces membranes de type ionique ne transportent que des ions oxygène et nécessitent l'application d'un champ électrique extérieur pour maintenir un flux d'électron à travers la membrane et ainsi le processus d'ionisation-désionisation.

Les membranes de conduction mixte ionique-électronique, notamment les membranes monophasiques constituées d'oxydes multimétalliques, sont en revanche capables de transporter à la fois des ions oxygène et des électrons sans qu'il soit nécessaire d'appliquer un champ électrique extérieur.

La force motrice permettant le transport de l'oxygène dans les membranes de conduction mixte se fonde sur une différence de pression partielle de O₂ appliquée de part et d'autre la membrane. De par sa structure non poreuse, la membrane empêche toute molécule de gaz de traverser directement. Seuls les ions oxygène peuvent migrer sélectivement. La dissociation et l'ionisation de l'oxygène se produit au contact de la surface où la pression partielle est la plus élevée (cathode), par capture d'électrons. Le flux de charges des ions oxygène O²⁻ est compensé par un flux simultané d'électrons dans le sens opposé. Lorsque les ions oxygènes atteignent le côté de faible pression partielle d'oxygène, soit le côté du perméat (anode), les ions oxygène cèdent leurs électrons et se recombinent pour régénérer de l'oxygène moléculaire libéré dans le courant de perméation.

Ces membranes, utilisées à titre de réacteurs membranaires pour la déshydrogénation oxydante de l'éthane transportent sélectivement de l'oxygène jusqu'à l'anode où ils réagissent rapidement avec l'éthane pour former l'éthylène.

Le transport de l'oxygène à travers ces membranes de conduction est un processus contrôlé qui dépend essentiellement de deux facteurs : les limitations cinétiques de surface et les limitations de diffusion volumique.

Les limitations cinétiques de surface sont liées à la multitude d'étapes mises en jeu lors de la conversion des molécules d'oxygène en phase gazeuse dans le courant de charge en ions oxygène mobiles et, inversement, des ions oxygène mobiles en molécules d'oxygène du côté du perméat de la membrane de conduction ou bien lors de la réaction des ions oxygène avec le gaz réactant.

Les limitations de diffusion volumique sont liées à la diffusion des ions oxygène et électrons à l'intérieur de la membrane.

Ces deux facteurs limitants dépendent notamment du gradient de pression partielle en O₂ de part et d'autre de la membrane, de la température opérationnelle et de l'épaisseur de la membrane.

Des travaux visant à optimiser les performances de ces membranes, utiles notamment à titre de réacteurs membranaires pour la déshydrogénation oxydante de l'éthane en éthylène, ont été menés.

Une première approche consiste à rechercher des compositions d'oxyde de conduction mixtes multimétalliques possédant de bonnes propriétés de transport de l'oxygène intrinsèques.

EP 0 766 330 décrit un élément pour utilisation dans des cellules électrochimiques.

Le document Haihui Wang et al., Chem. Comm., 2002, 1468-1469*,* décrit une réaction de déshydrogénation oxydante de l'éthane en éthylène dans un réacteur membranaire constitué de Ba_{0,5} Sr_{0,5} Co_{0,8} Fe_{0,2} O_{3-δ}.

L'éthylène est obtenu avec un rendement de 15 %, un taux de conversion de 18 %, une sélectivité de 90 % à 650°C et un flux de perméation d'oxygène de 0,36 mL.min⁻¹ cm⁻².

Dans le document H. Wang et al. (Catalysis Letters, 2002, vol. 84, Nos 1-2, pp. 101-106*),* la réaction de déshydrogénation oxydante de l'éthane dans le réacteur membranaire Ba_{0,5}Sr_{0,5}Co_{0,8}Fe_{0,2}O_{3-δ} est étudiée à 800°C. Un rendement maximum de 67 % est obtenu avec une sélectivité vis-à-vis de l'éthylène, un taux de conversion et un flux de perméation de l'oxygène respectivement de 80 %, 82 % et de 1,6 mL cm⁻² min⁻¹.

Une autre approche consiste à réduire les limitations cinétiques de surface en associant un catalyseur favorisant, par exemple, la dissociation de l'oxygène gazeux en ions O²⁻ mobiles.

Ainsi, le document EP 0 663 231 (Air Products and Chemicals, Inc.) décrit une membrane comprenant une couche poreuse de conduction mixte d'oxyde multimétallique dont une surface est revêtue d'un catalyseur et l'autre est en contact avec une couche dense de conduction mixte multimétallique. Le catalyseur comprend un métal ou un oxyde métallique qui catalyse la dissociation des molécules d'oxygène en ions et/ou l'association des ions oxygènes en oxygène moléculaire.

Les deux membranes exemplifiées dans cette demande de brevet mettent en oeuvre un catalyseur à base de platine à raison de 10 mg/cm² de surface. L'utilisation d'un métal précieux de coût élevé en quantité non négligeable à titre de catalyseur rend improbable l'application de ces membranes à l'échelle industrielle.

Le document WO 99/21649 décrit un réacteur catalytique membranaire comprenant une zone d'oxydation et une zone de réduction séparées par une membrane imperméable aux gaz, possédant une surface d'oxydation en contact avec la zone d'oxydation et une surface de réduction en contact avec la zone de réduction, une couche de catalyseur adhérente sur la surface d'oxydation de la membrane et, éventuellement, un catalyseur tri-dimensionnel dans la zone d'oxydation. Cependant, les catalyseurs se présentent sous forme de couche ou de revêtement continus.

De plus, seuls les catalyseurs La_{0,8} Sr_{0,2} MnO₃ et cermet de Ni sur La_{0,8} Sr_{0,2} MnO₃ sont réellement envisagés. Nulle part n'est fait mention de particules dispersées à base de magnésium ou de métaux nobles.

Ces membranes sont difficiles à mettre en oeuvre et il a été tenté de mettre au point des membranes performantes, moins coûteuses, et surtout beaucoup plus faciles à mettre en oeuvre.

De façon surprenante, il a maintenant été découvert qu'en dispersant une faible quantité de catalyseur à base d'oxyde de magnésium ou de métaux nobles sous la forme de particules à la surface d'une membrane de conduction mixte d'oxyde multimétallique, il était possible d'obtenir des membranes peu coûteuses possédant des taux de conversion et de sélectivité élevés.

De façon plus spécifique, il a été montré que cette modification de la surface de la membrane permet d'augmenter de façon significative le flux d'oxygène et/ou le rendement catalytique, et/ou d'abaisser les conditions de températures opérationnelle. A titre d'exemple, les conditions de températures opérationnelles peuvent être diminuées de 10 à 20%, ce qui peut représenter une centaine de degrés.

Cette baisse de température présente de nombreux avantages. Outre la réduction du coût opérationnel que représente une diminution de température d'une centaine de degrés pour une production industrielle, celle-ci permet de diminuer sensiblement les problèmes de corrosion au niveau du réacteur, et offre également un plus large choix de matériaux pour la réalisation du réacteur lui-même.

Elle permet également d'envisager la mise en oeuvre d'un nombre plus important de réactions, notamment celles nécessitant des températures élevées, telles que l'oxydation partielle d'alcanes légers.

En fait, de façon particulièrement intéressante, il a été découvert que la mise en contact d'une membrane dense de conduction mixte d'oxyde multimétallique avec des particules dispersées à base d'oxyde de magnésium ou de métal noble modifie les propriétés redox de la membrane.

Sans vouloir se limiter à une quelconque théorie, on pense que les particules à base d'oxyde de magnésium ou de métaux nobles dispersées sur la surface d'oxydation (ou anode) de la membrane favoriseraient la recombinaison de ions oxygènes en O₂ puis la désorption des molécules de O₂.

De surcroît, la présence des particules dispersées à base d'oxyde de magnésium ou de métaux nobles sur l'une des surfaces de la membrane permet d'accroître la stabilité thermique de la membrane résultante. Les membranes se révèlent ainsi beaucoup plus stables, notamment vis-à-vis des contraintes mécaniques et thermiques auxquelles elles sont soumises dans les conditions opérationnelles, maintenant leur intégrité structurale sur une plus longue durée.

Un des buts de la présente invention est de proposer des membranes de conduction mixte d'oxydes multimétalliques modifiées en surface présentant un flux vis-à-vis de l'oxygène amélioré par rapport aux membranes nues.

Selon un aspect avantageux de l'invention, les membranes composites selon l'invention permettent une augmentation du flux de perméation de l'oxygène, généralement comprise entre 5 et 20%, et avantageusement de l'ordre de 30%.

Un autre but de la présente invention est de proposer des membranes présentant une activité catalytique élevée pour des températures opérationnelles réduites.

Au sens de la présente description, une « activité catalytique élevée» signifie une activité permettant d'atteindre des rendements supérieurs à 60%, de préférence supérieurs à 70%, et plus préférentiellement supérieurs à 80%. A titre illustratif, les membranes selon l'invention permettent de convertir l'éthane en éthylène avec un rendement supérieur à 65%.

Un autre but de la présente invention est de fournir un procédé de préparation de ces membranes. Avantageusement, celui-ci est facile à mettre en oeuvre, peu coûteux et aisément applicable à l'échelle industrielle, ce qui devrait favoriser l'application et le développement des réacteurs membranaires de l'invention à l'échelle industrielle.

Un autre but de la présente invention est de proposer des réacteurs catalytiques membranaires comprenant les membranes selon l'invention. De façon avantageuse, ces réacteurs membranaires permettent d'atteindre des taux de conversion et de sélectivité élevés.

Les membranes selon l'invention sont utiles dans une grande variété de procédés dont notamment la séparation d'oxygène d'un mélange gazeux ou l'oxydation des hydrocarbures légers.

De façon avantageuse, les membranes selon l'invention présentent un flux de perméation d'oxygène accru et permettent donc d'extraire l'oxygène d'un mélange gazeux avec un rendement élevé à température plus faible, comparativement aux membranes de conduction d'oxygène nues, c'est-à-dire dépourvues du dépôt de particules dispersées à base d'oxyde de magnésium ou de métaux nobles.

Notamment, les membranes selon l'invention sont particulièrement utiles à titre de réacteur membranaire pour la déshydrogénation oxydante de l'éthane, en ce qu'elles permettent d'obtenir des taux de conversion, des sélectivités et des rendements supérieurs à ceux décrits dans l'art antérieur.

Un autre but de la présente invention est donc de fournir un procédé de déshydrogénation oxydante de l'éthane en éthylène, lequel constitue une alternative intéressante au procédé de déshydrogénation thermique de l'éthane.

En effet, outre les taux de conversion et les sélectivités élevées, ce procédé permet avantageusement de pallier aux problèmes de cokéfaction du procédé de déshydrogénation thermique. De surcroît, la séparation de l'oxygène couplée à sa consommation dans la réaction de déshydrogénation oxydante produit un certain nombre d'avantages : la formation de produits secondaires oxygénés est limitée, l'air, à la pression atmosphérique notamment, peut être utilisé directement comme source d'oxygène sans qu'il soit nécessaire de procéder à une distillation cryogénique ou autre, et les conditions opérationnelles peuvent être contrôlées sans danger d'explosion pour l'installation.

Ces buts et d'autres sont atteints par l'invention qui vise, selon un premier aspect, une membrane de conduction d'oxygène, comprenant une membrane dense de conduction mixte d'oxyde multimétallique décrit dans les revendications, dont l'une des surfaces est revêtue par des particules dispersées à base d'oxyde de magnésium ou de métaux nobles.

Selon un premier objet, la présente invention concerne une membrane de conduction d'oxygène comprenant une membrane dense de conduction mixte d'oxyde multimétallique dont l'une des surfaces est revêtue par des particules dispersées à base d'oxyde de magnésium ou de métaux nobles, dans laquelle la membrane dense de conduction mixte comprend l'oxyde multimétallique de formule :

Baₓ Sr₁₋ₓ Co_{1-y} Fe_{y} O_{3-z}

dans laquelle
0 ≤ x ≤ 1 ;
0 ≤ y ≤ 1;
z est un nombre rendant la charge du composé neutre et explicitant la déficience en oxygène.

### MEMBRANES

### Membrane dense de conduction mixte d'oxydes multimétalliques

Le terme «dense», signifie, au sens de la présente description, une couche « imperméable aux fluides gazeux », i.e ne permettant pas le passage d'une quantité substantielle d'un fluide gazeux à travers la membrane. Dans certains cas, une perméabilité mineure vis-à-vis d'autres gaz que le O₂ peut être acceptable ou inévitable. C'est le cas par exemple lorsque la membrane comprend des défauts de structures ou encore quand l'étanchéité du réacteur est défaillante.

En règle générale, l'expression « membrane dense » au sens de la présente description englobe des membranes ayant une permsélectivité vis-à-vis des gaz supérieure à 30, et préférentiellement supérieure à 100.

Les membranes denses de conduction mixte d'oxyde multimétalliques transportent les anions oxygène et sont en ce sens perméables à l'oxygène. En revanche, elles sont imperméables à l'oxygène gazeux à proprement parler.

Par « conduction mixte », on comprend, au sens de la présente description, une couche dense capable de transporter à la fois des électrons et des ions oxygène, et ce, de façon sélective.

La couche dense de l'invention est formée d'un seul ou d'un mélange de deux ou plusieurs oxydes multimétalliques, chaque oxyde multimétallique comprenant un oxyde d'au moins deux métaux différents ou un mélange d'au moins deux oxydes métalliques différents, dans laquelle l'oxyde multimétallique présente une conductivité électronique et oxygène-ionique à des températures supérieures à environ 500°C. On peut parler aussi de solution solide d'oxyde multimétallique.

Par « température opérationnelle », on entend, au sens de la présente description, une température à laquelle la membrane selon l'invention présente une conductivité électronique et oxygène-ionique significative.

En règle générale, les températures opérationnelles sont comprises entre 500°C et 1100°C environ. La température spécifique, de même que les flux de gaz réactant et de gaz contenant de l'oxygène, sont optimisés en fonction de la membrane, des particules de catalyseur et de l'application envisagée. La température doit être suffisamment élevée pour favoriser le flux d'anion oxygène à travers la membrane sans toutefois altérer la membrane et les particules de catalyseur.

La couche dense de conduction mixte d'oxyde multimétallique de l'invention désigne donc une membrane présentant une conductivité électronique et oxygène-ionique et la capacité de séparer l'oxygène d'un mélange gazeux contenant de l'oxygène et un ou plusieurs autres composants volatiles au moyen desdites conductivités.

Selon un mode préférentiel, la couche dense est formée d'oxydes mixtes présentant une structure perovskite.

Les composés de type perovskite répondent à la formule ABO₃ dans laquelle A représente un ion d'un métal alcalin, alcalino-terreux ou de terre rare et B un ion d'un métal de transition. Idéalement, ils présentent une structure cristalline cubique très stable, dans laquelle les ions A sont positionnés aux sommets de la maille cristalline cubique, les ions oxygène sont situés au centre de chaque face du cube et forment un octaèdre BO₆ avec B placé au centre du cube. Les structures perovskites idéales présentent généralement une somme des valences des ions A et B égale à 6, comme dans le modèle perovskite minérale, CaTiO₃.

La substitution d'un site A ou B par un autre alcalino-terreux ou cation métallique peut générer des sites vacants en oxygène. Ces sites vacants permettent le transport d'oxygène à travers le réseau cristallin.

Des exemples de structure perovskite en sont notamment les perovskites cubiques, les Brownmillerites, les phases Aurivillius ou perovskites lamellaires et analogues.

De préférence, les structures perovskites au sens de la présente description comprennent les structures ayant une phase perovskite supérieure à 90%, plus préférentiellement supérieure à 95 % et mieux encore supérieure à 98%.

La membrane dense de conduction mixte peut désigner un composé de formule générale :
A₁₋ₓ A'ₓ B_{1-y} B'_{y} O_{3-z}
dans laquelle
A et A', identiques ou différents, représentent chacun un ion métallique ou un métal alcalino-terreux ou d'un métal choisi dans la série des lanthanides ;
B et B', identiques ou différents, représentent chacun un ion métallique et/ou un mélange d'ions métalliques dans lequel le métal est choisi parmi les métaux de transition ;
0≤x≤1;
0≤y≤1 ;
z est un nombre rendant la charge du composé neutre et explicitant la déficience en oxygène ; z est ainsi fonction de la nature de A, A', B, B', de x, y et de la température.

A et A' peuvent être identiques ou différents, représenter La, Ca, Sr, et/ou Ba, ou bien encore Ba et/ou Sr.

A et A' peuvent être différents.

A peut représenter Ba. A' peut représenter Sr.

B et B' peuvent être identiques ou différents, représenter Cr, Mn, Fe, Co, Ni et/ou Cu, ou bien encore Co et/ou Fe.

B et B' peuvent être différents.

B peut représenter Co.

B' peut représenter Fe.

De préférence, y est compris entre 0,2 et 0,8, et mieux encore entre 0,6 et 0,8.

Les oxydes de conduction mixtes préférés sont choisis parmi

BaₓSr₁₋ₓCo_{y}Fe_{1-y}O_{3-z}

où x, y et z sont tels que définis précédemment.
Selon un aspect préféré de l'invention, x est compris entre 0,2 et 0,8, et préférentiellement entre 0,4 et 0,6.
De préférence, y est compris entre 0,2 et 0,8, et mieux encore entre 0,6 et 0,8. Dans ce cadre, il est particulièrement préféré que x soit compris entre 0,4 et 0,6 et y entre 0,6 et 0,8.

Plus préférentiellement, la membrane dense comprend l'oxyde multimétallique Ba_{0,5} Sr_{0,5} Co_{0,8} Fe_{0,2} O_{3-z}.

La membrane dense doit posséder une épaisseur suffisante pour présenter une bonne imperméabilité aux gaz et une bonne stabilité mécanique vis-à-vis des contraintes opératoires en réacteur.

Cependant, elle ne doit pas présenter une épaisseur excessive pour ne pas limiter le flux des ions oxygène à travers la membrane. En effet, de façon générale, le flux des ions oxygène augmente lorsque l'épaisseur de la membrane dense diminue jusqu'à une valeur critique à laquelle les cinétiques de désorption des ions oxygène deviennent limitantes.

L'épaisseur minimum de la membrane dense non supportée est de préférence d'environ 0,5 mm. L'épaisseur maximum de la membrane dense de conduction mixte est de préférence d'environ 10 mm, plus préférentiellement d'environ 2 mm, et mieux encore d'environ 1 mm.

De préférence, les membranes selon l'invention présentent une épaisseur comprise entre 0,5 et 2 mm, plus préférentiellement entre 0,9 et 1,6 mm et mieux encore entre 0,9 et 1,1 mm.

Classiquement, la membrane dense de conduction mixte présente un flux de perméation vis-à-vis de l'oxygène supérieur à 7.4x10⁻⁹ moles cm⁻²s⁻¹, mieux encore supérieur à 3.7x10⁻⁹, à une différence de pression partielle en oxygène d'environ 200 hPa dans les conditions de température opérationnelles optimales.

A titre indicatif, les membranes décrites dans la littérature possèdent généralement une épaisseur de 1 à 2 mm et se caractérisent par un flux de perméation d'oxygène variant de 10⁻⁷ à 10⁻⁹ moles cm⁻² s⁻¹, à une différence de pression partielle en oxygène d'environ 200 hPa.

### Dépôt catalytique

L'expression « revêtue » signifie, au sens de la présente description, qu'au moins l'une des surfaces de la membrane dense est directement en contact avec les particules à base d'oxyde de magnésium ou de métaux nobles.

Par « particules », on entend, au sens de la présente description, des particules de morphologie sphérique ou anisotrope, pouvant présenter une structure cristalline ou non, et possédant un diamètre moyen inférieur ou égal à 3 µm, avec une distribution granulométrique étroite de préférence.

Par « dispersées », on entend, au sens de la présente description, une distribution de particules discrètes réparties de façon aléatoire ou organisée, formant un dépôt discontinu à la surface de la membrane, étant entendu que les zones couvertes par les particules représentent entre 10 et 80% de la surface de la membrane.

Il est à noter que les zones de ladite surface de la membrane non couverte par des particules à base d'oxyde de magnésium ou de métaux nobles sont nues, c'est-à-dire qu'elles sont directement exposées aux gaz réactants ou au gaz contenant de l'oxygène. En d'autres termes, cette surface modifiée ne comporte pas d'autre revêtement.

La dispersion de particules à base d'oxyde de magnésium ou de métaux nobles directement en contact avec une membrane dense permet en effet la création de « points triples », i. e. d'augmenter la surface de contact entre le catalyseur, c'est-à-dire les particules à base d'oxyde de magnésium ou de métaux nobles, le gaz réactant et l'oxygène « actif » (02) à la surface de la membrane.

De façon avantageuse, ce type de dépôt permet de plus de limiter les phénomènes de réabsorption du produit formé sur la surface d'oxydation par le catalyseur pouvant conduire à sa dégradation éventuelle. Ceci se traduit donc de façon intéressante par une amélioration de la sélectivité, en particulier dans le cas où les produits générés sont sensibles à l'action du catalyseur, comme par exemple dans le cas de la déshydrogénation oxydante de l'éthane en éthylène.

Au sens de la présente description, le terme « particules à base d'oxyde de magnésium » désigne, au sens large, toute particule constituée en tout ou partie par un oxyde de magnésium, l'oxyde de magnésium au sein du matériau étant présent sous au moins une des formes suivantes :
- sous la forme d'oxyde de magnésium de formule MgO ; et/ou
- sous la forme d'oxyde de magnésium dit « dopé », à savoir un oxyde de magnésium intégrant, des cations métalliques d'insertion ou de substitution.

Dans un oxyde de magnésium dopé, les cations magnésium restent en général majoritaires, c'est-à-dire que le ratio molaire de la quantité de magnésium rapportée à la quantité totale de cations dans l'oxyde de magnésium dopé reste en général supérieur à 50% en mole, le plus souvent supérieur à 70% et, dans la plupart des cas, supérieur à 90% en mole.

De préférence, l'oxyde de magnésium est dopé par un métal de transition, préférentiellement choisi parmi le vanadium.

Selon un aspect particulièrement préféré, l'oxyde de magnésium est dopé par le vanadium, de préférence par 0,5 à 10 % en poids de vanadium, mieux encore par 5 % en poids de vanadium.

Par « particules de métaux nobles », on entend des particules constituées essentiellement d'un métal noble choisi parmi le Pd, le Pt, le Rh, l'Au, le Ru, l'Ag et l'Ir ou d'un alliage de ceux-ci, le métal noble étant sous forme réduite ou oxydé en tout ou partie. Parmi les métaux nobles, le Pd est particulièrement préféré.

De préférence, les particules à base d'oxyde de magnésium ou de métaux nobles sont dispersées selon une densité comprise entre respectivement 1.10⁵ et 1.10⁷ particules par cm² et 1.10¹² et 5.10¹² particules par cm².

Selon un mode préférentiel, le dépôt discontinu formé par les particules à base d'oxyde de magnésium ou de métaux nobles possède une surface spécifique variant de 30 à 50 m²/g, de préférence entre 40 et 50 m²/g à une température opérationnelle de 800°C.

A titre préféré, les particules à base d'oxyde de magnésium ou de métaux nobles possèdent un diamètre moyen compris entre 5 nm et 3000 nm, plus préférentiellement entre 5 et 50 nm et mieux encore entre 5 et 10 nm.

Cette taille moyenne de particules permet avantageusement de disposer d'une surface spécifique importante, i. e. d'augmenter la surface de contact entre le gaz et la membrane modifiée par unité de volume.

Le pourcentage en poids du dépôt formé par les particules à base d'oxyde de magnésium ou de métaux nobles rapporté au poids de la couche dense de conduction mixte peut varier entre environ 0,01 et 1%, de préférence entre 0,01 et 0,5%, plus préférentiellement entre 0,01 et 0,1 %.

### PROCEDE DE PREPARATION DES MEMBRANES SELON L'INVENTION

### Catalyseur à base d'oxyde de magnésium

Selon un deuxième objet, l'invention concerne les procédés de préparation des membranes de conduction d'oxygène définies ci-dessus.

Les particules à base d'oxyde de magnésium peuvent être dispersées et déposées par voie chimique à la surface de la couche dense d'oxyde multimétallique en utilisant des techniques conventionnelles telles que le dépôt de suspensions colloïdale encore appelé procédé sol-gel, ou le dépôt en phase vapeur.

Selon un mode de réalisation particulier, les membranes revêtues de particules dispersées à base d'oxyde de magnésium selon l'invention sont préparées selon les méthodes dites "sol-gel" connues de l'homme du métier.

Pour plus de précision à ce sujet, on pourra se référer notamment à la thèse de Laure Albaric, Université de Montpellier II, 1999.

Ce procédé comprend les étapes consistant à :
a. fournir une membrane de conduction mixte d'oxyde multimétallique telle que définie précédemment ;
b. préparer une suspension colloïdale à base d'oxyde de magnésium dans un solvant organique ;
c. mettre en contact la suspension obtenue avec la membrane dense de conduction mixte ; et
d. calciner la membrane obtenue.

La suspension colloïdale à base d'oxyde de magnésium peut être préparée selon les méthodes dites "sol-gel" connues de l'homme du métier.

La préparation de suspension colloïdale comprend ainsi la dissolution du précurseur des particules à base d'oxyde de magnésium dans le solvant organique, dans les proportions désirées. La concentration de la solution de précurseur est fixée selon le revêtement que l'on souhaite obtenir, et l'agitation se fait généralement à température ambiante jusqu'à l'obtention d'une suspension colloïdale.

De préférence, les précurseurs sont distillés avant leur utilisation de manière à éliminer les traces d'eau éventuelles et les espèces condensées.

A titre d'exemple de précurseurs de particules à base d'oxyde de magnésium, on peut notamment citer les alkoxydes, les hydroxydes, les nitrates, les alkoxydes de magnésium, tel que le diméthoxyde de magnésium.

Selon un mode de réalisation préféré, l'oxyde de magnésium est dopé par du vanadium. Dans ce cas, la suspension colloïdale est préparée à partir d'un mélange de précurseurs à base de magnésium et de vanadium dans les proportions souhaitées. A titre préféré, on prépare une solution d'alkoxyde de magnésium et d'alkoxyde de vanadium, de préférence de diméthoxyde de magnésium et d'isopropoxyde de vanadium.

Les solvants organiques mis en oeuvre à l'étape b) sont ceux couramment utilisés pour la préparation de suspensions colloïdales et englobent notamment le méthanol, l'éthanol, le 2-propanol.

La suspension colloïdale obtenue à l'étape b) est ensuite déposée à la surface de la membrane dense de conduction mixte selon la technique connue du « spin-coating ». A ce sujet, on pourra notamment se référer à Hiromichi Sakamoto, Jianbei Qiu and Akio Makishima, Science and Technology of Advanced Materials, Volume 4, Issue 1, 2003, Pages 69-76*.* Cette technique consiste généralement à déposer quelques millilitres de suspension colloïdale par exemple, au moyen d'une seringue, à la surface de la membrane dense, et à répartir la solution colloïdale de façon homogène sur la surface de la membrane, en plaçant la membrane sur un support mis en rotation à une vitesse élevée.

Le dépôt est alors séché à l'air puis dans une étuve à 100°C pendant une durée suffisante pour éliminer le solvant.

La membrane résultante est ensuite calcinée à une température suffisante pour éliminer les résidus organiques, généralement à une température supérieure à 400°C, de préférence comprise entre 800° et 900°C et mieux encore entre 800° et 850°C, ce par quoi on obtient une membrane revêtue de particules dispersées à base d'oxyde de magnésium.

La membrane dense de conduction mixte mise en oeuvre selon ce procédé peut être préparée selon les méthodes classiques comprenant généralement :
- la dissolution dans l'eau des sels métalliques constituant l'oxyde de conduction mixte, dont notamment des nitrates et des chlorures, dans les proportions stoechiométriques désirées;
- la co-précipitation des sels à partir de la solution aqueuse par addition d'un acide organique, par exemple l'acide citrique ;
- l'isolement du co-précipité par filtration ou centrifugation ; et
- la calcination du co-précipité pour obtenir un oxyde de conduction mixte multimétallique ;
- le broyage de l'oxyde de conduction mixte sous forme de poudre ;
- le moulage de la poudre d'oxyde de conduction mixte, éventuellement en association avec des additifs, sous la forme désirée ; et
- le frittage de la poudre moulée pour obtenir un matériau céramique dense de conduction mixte.

Ces méthodes, bien connues de l'homme du métier conviennent à la préparation de la membrane dense de conduction mixte des membranes selon l'invention.

La méthode « sol-gel » est particulièrement appropriée pour l'obtention de particules de taille micrométrique ou submicrométrique et leur dispersion à la surface de la membrane. La taille des particules formées peut être évaluée une fois les particules déposées à la surface de la membrane par microscopie électronique à balayage et/ou physisorption.

### Catalyseur à base de métaux nobles

Les membranes revêtues de particules dispersées de métaux nobles selon l'invention peuvent être préparées selon le procédé consistant à :
a) fournir une membrane de conduction mixte d'oxyde multimétallique telle que définie précédemment ;
b) déposer les particules de métaux nobles par vaporisation laser.

Le principe de la technique de vaporisation laser est exposé notamment dans la référence bibliographique « Ion-scattering study and Monte Carlo simulations of surface segregation in Pd-Pt nanoclusters obtained by laser vaporization of the bulk alloys », J.-L. Rousset et al., Physical Review B, 15 July 1998, 11, vol. 58, N ° 4.

Avantageusement, cette technique permet le dépôt de particules métalliques de petites tailles avec une distribution étroite et homogène, une composition et une quantité contrôlées.

La taille et la dispersion ou densité des particules sur la surface de la membrane dense peuvent être déterminées par microscopie électronique à transmission ou/et par physisorbtion.

Selon un troisième objet, l'invention concerne les membranes susceptibles d'être obtenues selon l'un des procédés définis ci-dessus.

Les membranes selon l'invention peuvent être préparées sous différentes formes, appropriées à un modèle particulier de réacteur, dont notamment sous forme de disques ou de tubes.

Les membranes de conduction mixte selon l'invention sont avantageusement stables à des températures comprises entre 25°C et 1100°C.

### REACTEUR MEMBRANAIRE

Selon un quatrième objet, l'invention vise également un réacteur membranaire catalytique comprenant une zone d'oxydation et une zone de réduction séparées par une membrane selon l'invention.

Dans ce qui suit, la surface d'oxydation (anode) désigne la surface en contact avec la zone d'oxydation et la surface de réduction (cathode), celle en contact avec la zone de réduction.

La surface d'oxydation correspond, de préférence, à celle en contact avec les particules à base d'oxyde de magnésium ou de métaux nobles dispersées.

La surface de réduction est en contact avec le mélange gazeux contenant de l'oxygène. Elle correspond de préférence à la couche dense, éventuellement revêtue d'une couche catalytique et est le siège des réactions de dissociation de l'oxygène moléculaire en ions oxygène O²⁻.

Cette surface de réduction peut être éventuellement revêtue par un catalyseur favorisant la réduction d'espèces contenant de l'oxygène telles que O₂, NO₂, SO₂ pour générer des anions oxygène au niveau de la membrane. A titre d'exemple de tels catalyseurs, on peut citer notamment les métaux nobles.

### APPLICATION DU REACTEUR MEMBRANAIRE

Les membranes selon l'invention sont utiles à titre de réacteurs membranaires pour l'oxydation partielle ou totale d'espèces réduites, en particulier des hydrocarbures.

Selon un cinquième objet, l'invention concerne un procédé pour oxyder un gaz réactant comprenant :
i) la mise en oeuvre d'un réacteur membranaire selon l'invention ;
ii) l'introduction du gaz réactant dans la zone d'oxydation ;
iii) l'introduction du gaz contenant de l'oxygène dans la zone de réduction ;
iv) le chauffage de la membrane séparant lesdites zones d'oxydation et de réduction à une température opérationnelle pour réduire le gaz contenant de l'oxygène, transporter les ions oxygène vers la zone d'oxydation et oxyder le gaz réactant.

De préférence, la température opérationnelle est comprise entre 500 et 800 °C.

A titre préféré, ce procédé est mis en oeuvre pour une oxydation partielle du gaz réactant, telle qu'une conversion du méthane en monoxyde de carbone et hydrogène, ou une déshydrogénation oxydante d'hydrocarbures, ou une oxydation ménagée d'hydrocarbures en molécules oxygénées.

Selon un mode de réalisation préféré, le gaz réactant est un hydrocarbure léger qui est oxydé en alcène.

Par « hydrocarbure léger », on entend au sens de la présente description des alcanes volatiles possédant de 1 à 6 atomes de carbone, dont notamment le méthane, l'éthane, le propane et les butanes.

De préférence, l'hydrocarbure léger est l'éthane ou le propane, de préférence l'éthane, lequel est oxydé en éthylène.

Selon un autre aspect avantageux, les membranes selon l'invention présentent une activité pour la déshydrogénation de l'éthane en éthylène supérieure à celle obtenue avec la membrane dense possédant une surface d'oxydation non modifiée par des particules dispersées à base d'oxyde de magnésium ou de métaux nobles.

Selon un autre objet, l'invention concerne l'utilisation des réacteurs membranaires pour récupérer de l'oxygène à partir d'un mélange gazeux contenant de l'oxygène.

Cette utilisation comprend notamment les étapes consistant en :
- l'introduction d'un mélange gazeux contenant de l'oxygène dans la zone de réduction, qui est séparée de la zone d'oxydation par la membrane selon l'invention, en établissant une différence de pression partielle positive entre les deux zones en produisant une pression partielle d'oxygène en excès dans le premier compartiment et/ou en produisant une pression partielle réduite dans le second compartiment ;
- la mise en contact du mélange contenant de l'oxygène avec la surface de réduction, à une température supérieure à environ 500°C pour convertir le mélange gazeux contenant de l'oxygène en courant enrichi d'oxygène (perméat), d'une part, et en courant d'oxygène appauvri, d'autre part ;
- la récupération dudit courant enrichi d'oxygène (perméat).

L'expression « mélange gazeux contenant de l'oxygène » désigne au sens large des gaz et des mélanges gazeux dont l'un des composants est l'oxygène ou un oxyde. Ces derniers peuvent être réduits sur la surface de réduction de la membrane selon l'invention. Cette expression couvre notamment les oxydes de carbone, d'azote de soufre (COₓ, NOₓ et SOₓ) et les mélanges gazeux comprenant un oxyde en présence d'un gaz inerte ou de tout autre gaz non réactif vis-à-vis de la membrane.

L'expression couvre donc également des mélanges d'oxygène moléculaire (O₂) dans d'autres gaz, par exemple l'oxygène de l'air ambiant, l'oxygène en présence d'un gaz inerte tel que He, Ar, etc.

Les membranes selon l'invention permettent ainsi de séparer l'oxygène à partir de mélanges gazeux contenant de l'oxygène et d'autres gaz tels que CO₂, H₂O et hydrocarbures volatiles. La proportion d'oxygène présente dans de tels mélanges gazeux est comprise généralement entre 0,01 % et 50 vol. %.

Selon un mode préférentiel, le mélange gazeux contenant de l'oxygène est l'air atmosphérique.

De façon particulièrement intéressante, la présence des particules dispersées à base d'oxyde de magnésium ou de métaux nobles sur les membranes de l'invention permet d'augmenter de 20% en moyenne la quantité d'oxygène récupérée.

### FIGURES

Figure 1 : Schéma d'un réacteur membranaire haute température.
Figure 2 : Aire spécifique du catalyseur VMgO en fonction de la température.
Figure 3 : Flux de perméation d'une membrane Ba_{0,5} Sr_{0,5} Co_{0,8} Fe_{0,2} O_{3-δ} revêtue de particules de MgO selon l'exemple 2 en fonction du temps à 725°C.
Figure 4 : Rendement en éthylène en fonction de la température obtenu avec une membrane Ba_{0,5} Sr_{0,5} Co_{0,8} Fe_{0,2} O_{3-δ} revêtue de particules de VMgO selon l'exemple 1.
Figure 5 : Sélectivité de la réaction de déshydrogénation oxydante en fonction de la température. Conditions : Zone de réduction, F_{AIR}=50mL/min, P_{Air}=1.2bars ; Zone d'oxydation, F_{C2H6/He}=37mL/min, P_{C2H6}=0.25 atm.
   Légende : Sethy, SCO₂, SCO, SCH₄ représentent les sélectivités en éthylène, en dioxyde de carbone, en monoxyde de carbone et en méthane respectivement.
Figure 6 : Rendement de la réaction de déshydrogénation oxydante en éthylène en fonction de la pression partielle d'éthane. Conditions :
   Température=775°C. Zone de réduction, F_{AIR}=50mL/min, P_{Air}=1.2bars ; Zone d'oxydation, F_{C2H6/He}=37mL/min.
Figure 7 : Rendement de la réaction de déshydrogénation oxydante en éthylène en fonction de la température. Conditions : Zone de réduction, F_{AIR}=50mL/min, P_{Air}=1.2bars ; Zone d'oxydation, F_{C2H6/He}=37mL/min, P_{C2H6}= 0.25 atm.
Figure 8 : Rendement de la réaction de déshydrogénation oxydante en éthylène en fonction de la température avec différents catalyseurs. Conditions:
   Zone de réduction, F_{AIR}=50mL/min, P_{Air}=1.2bars ; Zone d'oxydation, F_{C2H6/He}=37mL/min, P_{C2H6}=0.25 atm.
Figure 9 : Rendement de la réaction de déshydrogénation oxydante avec une membrane selon l'exemple 2 en fonction de la température. Conditions :
   Zone de réduction, F_{AIR}=50mL/min, P_{Air}=1.2bars; Zone d'oxydation, F_{C3H8/He}=37mL/min, P_{C3H8}=0.20 atm.
      Légende : Y alcène, Y éthy, Y propy représentent les rendements en alcène, éthylène et propylène respectivement.
Figure 10 : Rendement de la réaction de déshydrogénation oxydante avec une membrane selon l'exemple 2 en fonction de la pression partielle de propane. Conditions : Température=700°C. Zone de réduction, F_{AIR}=50mL/min, P_{Air}=1.2bars ; Zone d'oxydation, F_{C3H8/He}=34mL/min.
   Légende : Y éthy, Y propy représentent les rendements en éthylène et propylène respectivement.
Figure 11 : Rendement de la réaction de déshydrogénation oxydante en fonction de la température avec une membrane selon l'exemple 3. Conditions :
   Zone de réduction, F_{AIR}=50mL/min, P_{Air}=1.2bars; Zone d'oxydation, F_{C3H8/He}=37mL/min, P_{C3H8}=0.20 atm.
Figure 12 : Visualisation de la surface de la membrane revêtue de particules de VMgO selon l'exemple 1 dispersées par microscopie électronique à balayage (Echelle : 10 µm/cm).
Figure 13 : Visualisation de la surface de la membrane revêtue de particules de Pd selon l'exemple 3 dispersées par microscopie électronique à transmission (Echelle : 25 µm/cm).

### EXEMPLES

### Abréviations

HC : hydrocarbure
He : hélium
C₂H₆ : éthane
C₂H₄ : éthylène
C₃H₈ : propane
F_{AIR}, F_{C2H6} : flux d'air ou d'éthane respectivement

### Matériels et méthodes

Les réactifs suivants ont été utilisés :
- Ba(NO₃)₂ Sr(NO₃)₂, Co(NO₃)₃.6H₂O Fe(NO₃)₂.6H₂O, EDTA, acide citrique (Sigma Aldrich®)
- Mg(OMe)₂ (9,34 g de Mg/L dans le méthanol) (Epichem®)
- VO(OCH₂CH2CH3)₃ (Aldrich®).

Les poudres obtenues ont été caractérisées par analyse élémentaire et par diffraction aux rayons X et par la méthode BET.

Les spectres de diffraction X ont été effectués avec un appareil système Brücker® D5005 dans l'intervalle 20 allant de 3°à 80°, un pas de 0,02°, un temps de 1 s et une radiation de Cu K_{α1+α2} = 1,54184 Å.

La composition élémentaire a été déterminée par ICP-OES avec un appareil spectroflamme.

Les microscopies électroniques à balayage ont été effectuées avec un appareil Hitach S-800. Les microscopies électroniques à transmission ont été effectuées avec un appareil JEOL 2010 - 200kV.

### Réacteur membranaire

### a) Description

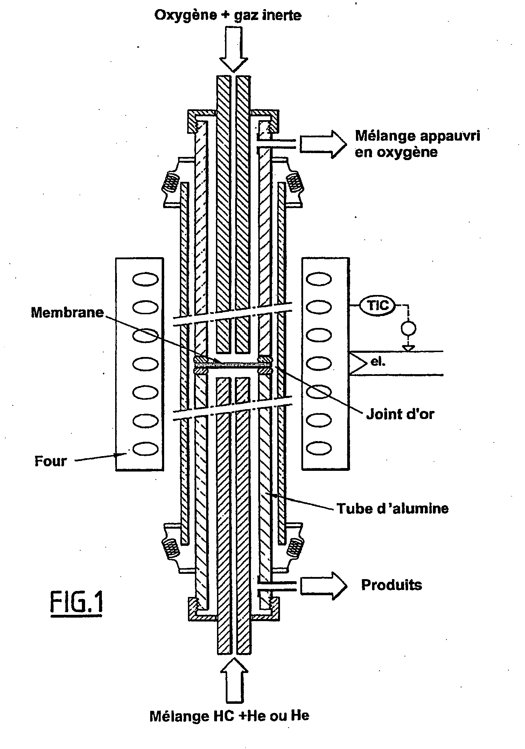

La déshydrogénation oxydante de l'éthane (DHOE) et la perméabilité vis-à-vis de l'oxygène ont été étudiées en utilisant le réacteur décrit Figure 1.

Les disques (d'environ 1 mm d'épaisseur) ont été scellés entre deux tubes denses d'alumine (OD 12 mm, ID 8 mm) en utilisant de l'or à titre d'agent scellant chimiquement inerte.

De plus, le côté de la paroi du disque a été largement couvert avec une colle à base d'or de manière à supprimer les contributions radiales du flux d'oxygène diffusant à travers la section active de 0,5 cm². La soudure a été réalisée au début des expériences en chauffant à une température de 800 °C pendant une nuit. Le côté en contact avec l'air a été alimenté avec une pression totale constante ajustée à 120 kPa et à un débit constant de 50 mL/min en utilisant un courant mixte de O₂ (air liquide) et de N₂ (N₂ sous forme air liquide évaporé, air liquide) contrôlé individuellement par des contrôleurs de flux massique (Brooks®, type 5850 TR). Le côté en contact avec le fuel a été alimenté avec un mélange d'éthane (air liquide) et d'hélium (air liquide) individuellement contrôlé par des contrôleurs de flux massique (Brooks®, type 5850 TR) dans le cas de le DHOE, et simplement par He dans le cas de la perméabilité vis-à-vis de l'oxygène. Deux capteurs de pression ont été installés, permettant de connaître en continu la pression totale sur chaque surface du réacteur.

La figure 1 représente un schéma d'un réacteur membranaire de perméation haute température avec des soudures en or.

### b) Analyse des gaz

Les gaz réactants (O₂, N₂, C₂H₆) et les gaz produits (H₂, CH₄, CO₂, C₂H₄, C₂H₆ et H₂O) ont été analysés par deux chromatographes en phase gazeuse (GC), tout deux connectés à une Chemstation HP, afin de collecter les données et permettre une analyse rapide. Le premier GC (HP 5890 Séries II) a été équipée avec un détecteur de conductivité thermique (DCT), et une colonne 13X permettant la séparation de O₂, N₂, CH₄, CO et H₂O. H₂, CH₄, CO₂, C₂H₄, C₂H₆ et H₂O ont été élués à partir d'une colonne Haye-Sep sur le second GC (Delsi Séries 200) équipé avec un DCT.

De plus, de l'argon a été introduit avec les réactifs afin de déterminer facilement une possible expansion du flux due à une augmentation du nombre de moles pendant le procédé de DHOE. L'équilibre en carbone est situé aux alentours de 4 %.

Des fuites de gaz liées à une mauvaise soudure ou à une densification incomplète de la membrane, si elles se produisaient, seraient ainsi détectées en suivant la concentration d'azote, ce qui permet de calculer la permsélectivité en O₂ du réacteur membranaire.

### Exemple 1 : Préparation d'une membrane dense Ba_{0,5} Sr_{0,5} Co_{0,8} Fe_{0,2} O_{3-δ} revêtue de particules de VMgO

### a) Préparation de la membrane dense Ba_{0,5} Sr_{0,5} CO_{0,8} Fe_{0,2} O_{3-δ}.

La poudre perovskite Ba_{0,5} Sr_{0,5} Co_{0,8} Fe_{0,2} O_{3-δ} (BSCFO) a été préparée en utilisant une variante de la méthode communément appelée "méthode citrate". Dans cette méthode, les quantités stoechiométriques de Ba(NO₃)₂ (2,61 g ; 0,5 eq), Sr(NO₃)₂ (2,11 g ; 0,5 eq), Co(NO₃)₂ 6H₂O (4.65 g ; 0,8 eq) et Fe(NO₃)₃ 6H₂O (4.05 mL d'une solution aqueuse à 0,988 mol/L ; 8,76 g ; 0,2 eq)(de pureté > 99,5%) ont été dissoutes dans 100 mL d'eau distillée suivie par l'addition d'EDTA et d'acide citrique avec un ratio molaire perovskite, EDTA et acide citrique égal à 1:1,5:3.

La solution de couleur violette obtenue a été chauffée à une température de 100°C jusqu'à l'obtention d'un matériau sous forme de gel formé par évaporation d'eau après environ trois heures. Le gel a ensuite été chauffé à 300 °C pendant trois heures. La mousse obtenue a ensuite été calcinée à 900 °C pendant quatre heures à l'air pour conduire à l'obtention d'une poudre de perovskite. Cette poudre a ensuite été moulue dans un mortier. Des disques de membrane nue ont été pressés à une pression de 140 MPa pendant une minute. La densification des disques a ensuite été réalisée par frittage à 1150°C pendant huit heures. L'épaisseur de la membrane est de 1 mm +/- 0.1 et la surface de 0.5 cm².

### b) Préparation et dépôt des particules de catalyseur VMgO

Les particules à base d'oxyde mixte de magnésium dopé par 10% en poids de vanadium ont été préparées à partir de précurseurs moléculaires par la méthode sol-gel. Celle-ci permet en effet de déposer des couches minces de pureté et de surface spécifique élevées.

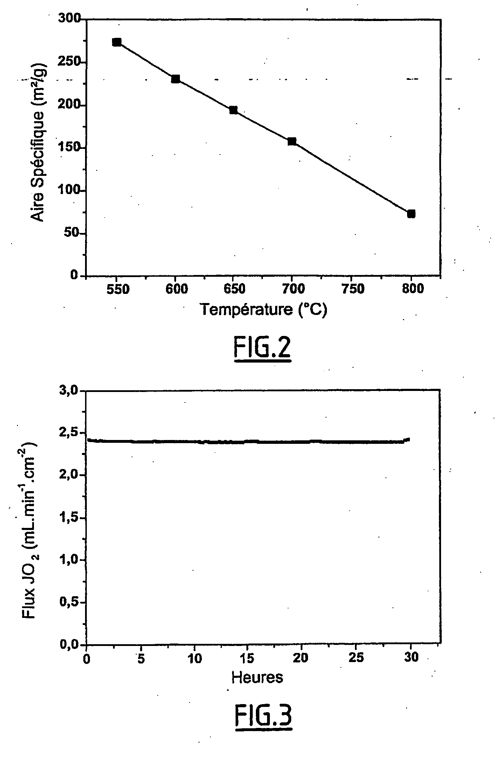

La figure 2 représente l'aire spécifique de VMgO en fonction de la température.

L'alkoxyde de magnésium utilisé (Mg(OMe)₂) (9.34g de Mg/L de méthanol) a été préparé par Epichem® et l'alkoxyde de vanadium (VO(OCH₂CH₃)₃ (208.8g deV/L de méthanol) est une solution commercialisée par Aldrich®. Les deux solutions ont été mélangées de façon à obtenir une solution dans laquelle le vanadium représente 10% en poids.

Quelques gouttes de la solution obtenue, correspondant à une quantité d'environ 1 mg de VMgO, ont été alors déposées sur la surface de la membrane par la technique du « spin-coating ». La membrane a ensuite été placée directement dans le réacteur à 850°C. On obtient ainsi des particules de VMgO de 2 µm, réparties selon une densité de [ 5.10⁶ part/cm² (à 10% près)] et recouvrant environ 40 % de la surface de la membrane (à 10 % près).

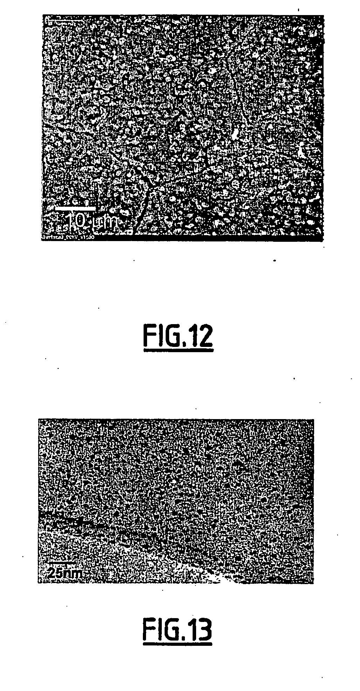

La Figure 12 représente la surface de la membrane en microscopie électronique (Echelle : 10 µm/cm).

### c) Caractérisation des poudres

La formation d'une structure perovskite pure a été vérifiée par diffraction aux rayons X.

La composition élémentaire a été déterminée par ICP-OES (spectroflamme) en analysant un échantillon dissout en chauffant de 250° à 300°C dans un mélange de H₂SO₄ et de HNO₃ après calcination.

**Tableau 1 : Composition cationique de la poudre BSCFO. Résultats de l'analyse ICP-OES.**

| Echantillon | Ba/at.-% | Sr/at.-% | Co/at.-% | Fe/at.-% |
|---|---|---|---|---|
| Batch 1 | 24,9 | 25,1 | 39,9 | 10,1 |
| Batch 2 | 25,0 | 25,0 | 40,0 | 10,0 |

Le tableau 1 montre que cette méthode assure une très bonne reproductibilité de la composition de la poudre.

### Exemple 2: Préparation d'une membrane dense BSFCO revêtue de particules de catalyseur MgO

La membrane dense de Ba_{0,5} Sr_{0,5} CO_{0,8} Fe_{0,2} O_{3-δ} est préparée conformément à celle de l'exemple 1.

Les particules à base d'oxyde de magnésium ont été préparées par la technique sol-gel à partir d'une solution méthanolique de Mg(OMe)₂ (Epichem®) à 9,34 g/L.

1 mg de la suspension colloïdale obtenue a été déposé sur la surface de la membrane par la technique du spin-coating. La membrane a ensuite été placée directement dans le réacteur à 850°C.

On obtient ainsi des particules de MgO de 2 µm de taille réparties selon une densité de [5.10⁶ part/cm² (à 10% près)] et couvrant environ 40 % de la surface de la membrane.

### Exemple 3 : Membrane dense BSFCO revêtue de particules de catalyseur Pd

La membrane dense mise en oeuvre est conforme à celle décrite dans l'exemple 1.

Les particules de Pd ont été déposées à la surface de la membrane dense selon la technique de la vaporisation laser. L'ablation laser d'un barreau de Pd de 99,99 % de pureté (Goodfellow) a été réalisée avec un laser YAG :Nd(BMI®) pulsé à 30 Hz et doublé en fréquence (532 nm).

La quantité de matière déposée à la surface de la membrane a été déterminée par une microbalance à quartz (INFICON®).

On obtient ainsi des particules de Pd de taille moyenne de 2-3 nm, réparties selon une densité de 4.10¹² part/cm², et couvrant environ 8 % de la membrane (à 2 % près).

La figure 13 représente la visualisation de la surface de la membrane revêtue de particules de Pd dispersées en microscopie électronique.

### Exemple 4 : Détermination de la vitesse de perméation d'oxygène

### Application de la membrane selon l'exemple 2 à la séparation d'oxygène

La stabilité de la membrane de l'exemple 2 vis-à-vis de la perméation de l'oxygène a été étudiée en enregistrant en continu le flux de perméation sous un gradient de pression partielle en oxygène constant pendant 30 heures.

Comme décrit dans la figure 3, un flux de perméation de l'oxygène stable de 2.4 mL/min.cm² est obtenu à 725°C. Ces résultats représentent un flux qui est 1.5 fois supérieur à celui correspondant à la membrane nue.

### Exemple 5 : Application de la membrane à la déshydrogénation oxydante de l'éthane

### a) Principales réactions possibles

| | |
|---|---|
| (1) C₂H₆ + ½ O₂ → C₂H₄ + H₂O | ΔrH °(750 °C) = - 93,927 kJ/mol |
| (2) C₂H₆ → C₂H₄ + H₂ | ΔrH °(750 °C) = 144,022 kJ/mol |
| (3) H₂ + ½ O₂ → H₂O | ΔrH °(750 °C) = - 240 kJ/mol |
| (4) C₂H₆ → CH₄ + H₂ + C | ΔrH °(750 °C) = 16,282 kJ/mol |
| (5) C + xO₂ → CO/CO₂ | |

### b) Calcul

Des expériences d'oxydation de l'éthane et du propane dans les réacteurs membranaires ont été réalisées à différentes températures 700-825°C, différents débits C₂H₆/He (35-65-100 mUmin) et aux pressions partielles de C₂H₆ de 0,25-0,44-0,55 atm du côté de la membrane en contact avec l'éthane (zone d'oxydation).

Tous les calculs ont été réalisés à partir de la pression partielle Pₓ, le flux total F°_{côté éthane}, le coefficient d'expansion du flux Cₑₓₚ et la surface de la membrane active S (dans l'hypothèse d'un comportement selon la loi pour un gaz idéal).

### c) Effet de la température

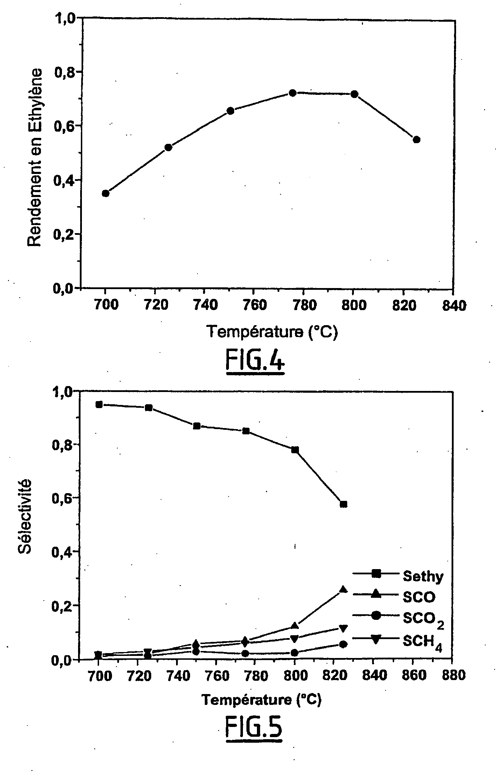

La figure 4 représente le rendement en éthylène en fonction de la température. Ce dernier augmente jusqu'à 775°C pour atteindre 75% (conversion=87%, sélectivité=87%). A 700°C, le faible rendement (35%) est dû à une mauvaise conversion (<40%) puisque la sélectivité est supérieure à 90% comme indiquée sur la figure 5. Par contre à 825 °C, le faible rendement (50%) est dû à une diminution de la sélectivité en éthylène (<60%) au profit du monoxyde de carbone.

### d) Effet de la pression partielle d'éthane

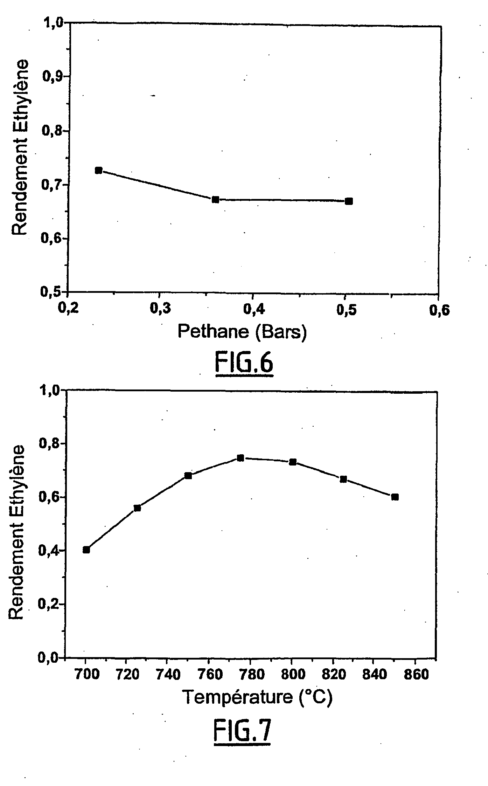

La figure 6 représente le rendement en éthylène en fonction de la pression partielle d'éthane à la température 'optimale' de 775°C. Aucune variation de rendement en fonction de la pression partielle d'éthane n'est observée. La sélectivité et la conversion sont également relativement constantes.

### Exemple 6 : Application des membranes selon les exemples 1 à 3 à la déshydrogénation oxydante de l'éthane

La figure 7 représente le rendement en éthylène de la membrane selon l'exemple 3 en fonction de la température. Ce dernier augmente jusqu'à 775°C pour atteindre un maximum de 75% qui correspond à un taux de conversion de 87% et un taux de sélectivité de 87%.

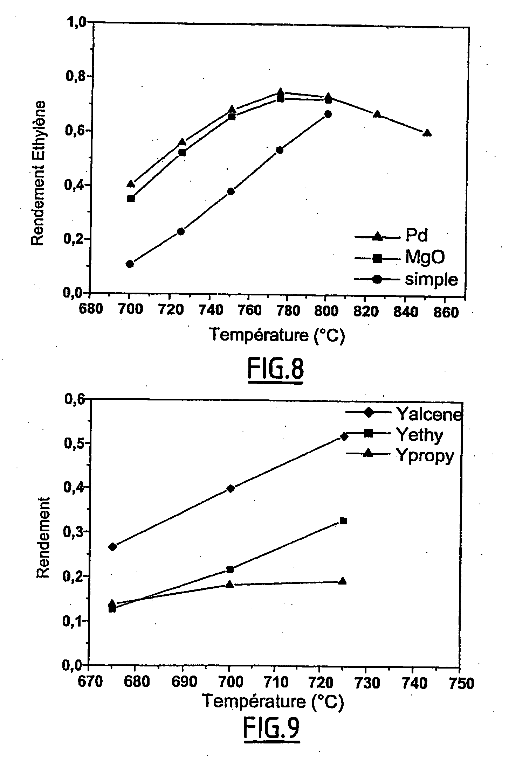

La Figure 8 compare les rendements d'éthane à différentes températures pour les membranes denses simples, c'est-à-dire nues, partiellement revêtues par des particules dispersées à base d'oxyde de magnésium (exemples 1 et 2) ou bien de Pd (exemple 3).

La présence de particules dispersées de Pd ou à base d'oxyde de magnésium permet d'atteindre un rendement donné à une température inférieure en moyenne de 75°C à celle des membranes denses.

La présence de particules dispersées de Pd ou à base d'oxyde de magnésium permet surtout d'obtenir des rendements supérieurs à ceux des membranes denses (Y=75%, C=87%, S=87%) à une température donnée.

Les rendements obtenues avec les particules de catalyseur à base d'oxyde de magnésium et de Pd sont comparables.

### Exemple 7 : Application de la membrane de l'exemple 2 à la déshydrogénation oxydante du propane

### a) effet de la température

La figure 9 indique les rendements en propylène, éthylène et alcène totale (i. e. propylène et éthylène) en fonction de la température. On obtient pour le propylène un rendement maximal de 19% (S=27.4%, C=69.7%) à 725°C. Ce faible rendement est dû principalement à une réaction secondaire de craquage du propane dans les conditions de température opérationnelles de la membrane. En effet, le produit principal obtenu est l'éthylène (S=47.2% à 725 °C), produit très valorisable.

### b) effet de la pression partielle de propane

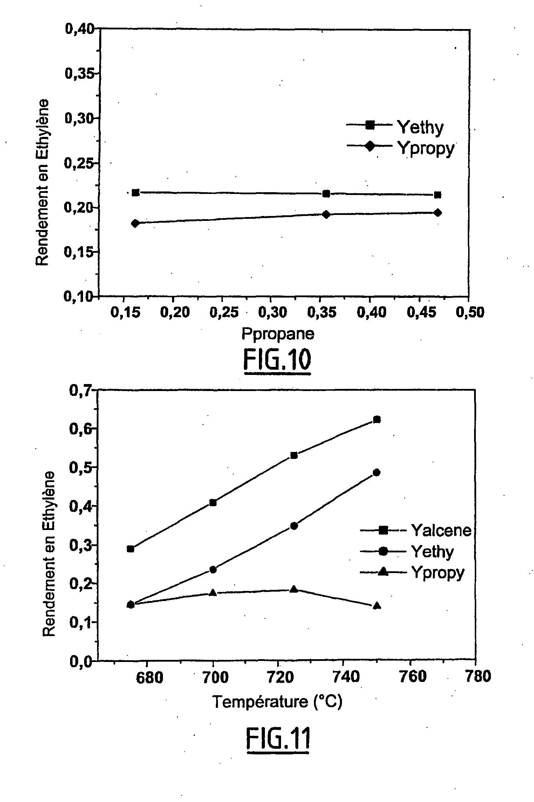

La figure 10 représente le rendement en propylène et ethylène à 700°C en fonction de la pression partielle en propane.

Le rendement en éthylène et en propylène reste constant pour une pression partielle en propane comprise entre 0,15 et 0,50 bars.

### Exemple 8 : Application de la membrane de l'exemple 3 à la déshydrogénation oxydante du propane

La figure 11 indique les rendements en propylène, éthylène et alcène (propylène et éthylène) en fonction de la température. On obtient pour le propylène un rendement maximal de 18.2% (S=25.9%, C=70.1 %) à 725°C.

### Exemple 9 : Etude comparative entre les membranes des exemples 1, 2 et 3 et les membranes de l'art antérieur

Les flux de perméation d'oxygène de différentes membranes ont été déterminées avec le réacteur décrit à la figure 1 dans les conditions indiquées dans la partie « Matériel et méthodes » ci-dessus.

Ces résultats montrent que la modification de l'une des surfaces de la membrane dense par des particules de métaux nobles ou à base d'oxyde de magnésium permet d'augmenter de façon significative le flux de perméation d'oxygène.

**Tableau 2**

| Membrane | T (°C) | Flux d'oxygène | Référence |
|---|---|---|---|
| Sans modification de surface | | Mol cm⁻²s⁻¹ | |
| Ba_{0,5}Sr_{0,5}Co_{0,8}Fe_{0,2}Oₓ | 800 | 6,69 E-07 | [1] |
| BaBi_{0,4}Co_{0,2}Fe_{0,4}O_{3-&} | 800 | 3,12 E-07 | [2] |
| La_{0,2}Sr_{0,8}Co_{0,2}Fe_{0,8}O_{3-&} | 800 | 1,12 E-07 | [3] |
| La_{0,6}Sr_{0,4}Co_{0,2}Fe_{0,8}O_{3-&} | 800 | 1,86 E-08 | [4] |
| La_{0,6}Sr_{0,4}CO_{0,8}Fe_{0,2}O_{3-&} | 800 | 5,20 E-08 | [5] |
| SrCo_{0,8}Fe_{0,2}O_{3-&} | 800 | 4,80 E-07 | [1] |
| BaCo_{0,4}Fe_{0,4}Zr_{0,2}O₃₋₁ | 800 | 3,35 E-07 | [7] |
| Bi₂V_{0,8}Mn_{0,2}O_{5,3} | 800 | 1,58 E-08 | [8] |
| Avec modification de surface Ba_{0,5}Sr_{0,5}CO_{0,8}Fe_{0,2}Oₓ+MgO | 800 | 1,00 E-06 | Exemple 2 |
| Ibid + Pd | 800 | 1,12 E-06 | Exemple 3 |
| Ibid + VMgO | 800 | 1,27 E-06 | Exemple 1 |

1. Shao, Z., et al., Investigation of the permeation behavior and stability of a Ba0.5Sr0.5Co0.8Fe0.2O3-[delta] oxygen membrane. Journal of Membrane Science, 2000. 172(1-2): p. 177-188.
2. Shao, Z., et al., Synthesis and oxygen permeation study of novel perovskite-type BaBixCo0.2Fe0.8-xO3-.delta. ceramic membranes. J. Membr. Sci., 2000. 164(1-2): p. 167-176.
3. Li, S.G., et al., Comparison of oxygen permeability and stability of perovskite type La(0.2)A(0.8)Co(0.2)Fe(0.8)O(3-delta) (A = Sr, Ba, Ca) membranes. Industrial and Engineering Chemistry Research, 1999. 38(8): p. 2963-2972.
4. Lee, S., et al., Oxygen-permeating property of LaSrBFeO3 (B=Co, Ga) perovskite membrane surface-modified by LaSrCoO3. Solid State lonics, 2003. 158(3-4): p. 287-296.
5. Teraoka, Y., et al., Catalytic effects in oxygen permeation through mixed-conductive LSCF perovskite membranes. Solid State lonics, 2002. 152-153: p. 681-687.
6. Qiu, L., Lee T. H., Oxygen permeation studies of SrCo0.8Fe0.2O3-&. Solid State lonics, 1995. 76: p. 321-329.
7. Tong, J., et al., Investigation of ideal zirconium-doped perovskite-type ceramic membrane materials for oxygen separation. Journal of Membrane Science, 2002. 203(1-2): p. 175-189.
8. Yang, Y.L., Qiu L. Jacobson, Manganese doped bismuth vanadate solid electrolytes: oxygen permeation in Bi2V0.8Mn0.2O5.3. Journal of Materials Chemistry, 1997. 7: p. 937-941.

## Revendications

1. Membrane de conduction d'oxygène comprenant une membrane dense de conduction mixte d'oxyde multimétallique dont l'une des surfaces est revêtue par des particules dispersées à base d'oxyde de magnésium ou de métaux nobles, dans laquelle la membrane dense de conduction mixte comprend l'oxyde multimétallique de formule :
Baₓ Sr₁₋ₓ Co_{1-y} Fe_{y} O_{3-z}
dans laquelle
0≤x≤1;
0≤y≤1;
z est un nombre rendant la charge du composé neutre et explicitant la déficience en oxygène.

2. Membrane selon la revendication 1, dans laquelle les particules à base d'oxyde de magnésium et/ou de métaux nobles ont un diamètre compris entre 5 et 3000 nm.

3. Membrane selon l'une quelconque des revendications 1 ou 2, dans laquelle la membrane dense de conduction mixte d'oxyde multimétallique présente une structure perovskite.

4. Membrane selon la revendication 1 dans laquelle l'oxyde multimétallique comprend le Ba_{0,5} Sr_{0,5} Co_{0,8} Fe_{0,2} O_{3-z}.

5. Membrane selon l'une quelconque des revendications précédentes, dans laquelle la membrane dense de conduction mixte d'oxyde multimétallique a une épaisseur comprise entre 0.5 et 10 mm.

6. Membrane selon l'une quelconque des revendications précédentes, dans laquelle les particules à base d'oxyde de magnésium ou de métaux nobles représentent 0,01 à 0,1 % en poids de la membrane dense.

7. Membrane selon l'une quelconque des revendications précédentes, dans laquelle les particules sont à base d'oxyde de magnésium.

8. Membrane selon la revendication 7, dans laquelle les particules à base d'oxyde de magnésium sont dopées par du vanadium.

9. Membrane selon l'une quelconque des revendications 1 à 6, dans laquelle les particules sont des particules de métaux nobles ou d'alliages de ceux-ci.

10. Membrane selon la revendication 9, dans laquelle les métaux nobles sont choisis parmi le Pd, le Pt, le Rh, l'Ag, l'Au, le Ru et l'Ir.

11. Procédé de préparation des membranes de conduction d'oxygène telles que définies selon les revendications 1 à 8, comprenant les étapes consistant à :
a. fournir une membrane dense de conduction mixte telle que définie dans les revendications 1 et 3 à 5 ;
b. préparer une suspension colloïdale à base d'oxyde de magnésium dans un solvant organique ;
c. mettre en contact la suspension obtenue avec la membrane dense de conduction mixte ; et
d. calciner la membrane obtenue.

12. Procédé de préparation des membranes de conduction d'oxygène telles que définis dans les revendications 1 à 6 et 9 à 10, comprenant les étapes consistant à :
a. fournir une membrane dense d'oxyde multimétallique telle que définie dans les revendications 1 et 3 à 5 ;
b. déposer les particules de métaux nobles ou d'alliages de ceux-ci par vaporisation laser.

13. Membrane de conduction d'oxygène susceptible d'être obtenue par le procédé selon l'une quelconque des revendications 11 et 12.

14. Réacteur membranaire comprenant une zone d'oxydation et une zone de réduction séparées par une membrane de conduction d'oxygène telle que définie dans l'une quelconque des revendications 1 à 10 ou 13.

15. Réacteur membranaire selon la revendication 14, dans lequel la zone d'oxydation est au contact de la surface de la membrane revêtue par des particules dispersées à base d'oxyde de magnésium ou de métaux nobles.

16. Procédé d'oxydation d'un gaz réactant comprenant :
i) la mise en oeuvre d'un réacteur membranaire selon les revendications 14 ou 15 ;
ii) l'introduction du gaz réactant dans la zone d'oxydation ;
iii) l'introduction du gaz contenant de l'oxygène dans la zone de réduction ;
iv) le chauffage de la membrane séparant lesdites zones d'oxydation et de réduction à une température opérationnelle.

17. Procédé selon la revendication 16, dans lequel le gaz réactant est un hydrocarbure léger qui est oxydé en alcène.

18. Procédé selon la revendication 17, dans lequel l'hydrocarbure léger est l'éthane qui est oxydé en éthylène.

19. Utilisation d'un réacteur membranaire selon les revendications 14 ou 15 pour récupérer de l'oxygène à partir d'un mélange gazeux contenant de l'oxygène.

## Patentansprüche

1. Sauerstoff leitende Membran, eine dichte gemischtleitende Membran aus Mehrmetalloxid umfassend, deren eine Oberfläche mit verteilten Partikeln auf der Basis von Magnesiumoxid oder Edelmetallen beschichtet ist, in der die dichte gemischtleitende Membran das Mehrmetalloxid der Formel
BaₓSr₁₋ₓCo_{1-y}Fe_{y}O_{3-z}
umfasst, in der
0 ≤ x ≤ 1,
0 ≤ y ≤ 1,
z eine Zahl ist, die die Ladung der Verbindung ausgleicht und den Sauerstoffmangel ausdrückt.

2. Membran nach Patentanspruch 1, in der die Partikel auf der Basis von Magnesiumoxid und/oder Edelmetallen einen Durchmesser zwischen 5 und 3000 nm haben.

3. Membran nach irgendeinem der Patentansprüche 1 oder 2, in der die dichte gemischtleitende Membran aus Mehrmetalloxid eine Perowskit-Struktur aufweist.

4. Membran nach Patentanspruch 1, in der das Mehrmetalloxid Ba_{0,5}Sr_{0,5}Co_{0,8}Fe_{0,2}O_{3-z} enthält.

5. Membran nach irgendeinem der vorangehenden Patentansprüche, in der die dichte gemischtleitende Membran aus Mehrmetalloxid eine Dicke zwischen 0,5 und 10 mm hat.

6. Membran nach irgendeinem der vorangehenden Patentansprüche, in der die Partikel auf der Basis von Magnesiumoxid oder Edelmetallen 0,01 bis 0,1 Gewichtsprozent der dichten Membran ausmachen.

7. Membran nach irgendeinem der vorangehenden Patentansprüche, in der die Partikel im Wesentlichen aus Magnesiumoxid bestehen.

8. Membran nach Patentanspruch 7, in der die Partikel auf der Basis von Magnesiumoxid mit Vanadium dotiert sind.

9. Membran nach irgendeinem der Patentansprüche 1 bis 6, in der die Partikel aus Edelmetallen oder deren Legierungen bestehen.

10. Membran nach Patentanspruch 9, in der die Edelmetalle unter Pd, Pt, Rh, Ag, Au, Ru und Ir ausgewählt wurden.

11. Verfahren zur Herstellung der Sauerstoff leitenden Membranen, wie sie in den Patentansprüchen 1 bis 8 definiert sind, folgende Schritte umfassend:
a. Bereitstellen einer dichten gemischtleitenden Membran, wie sie in den Patentansprüchen 1 und 3 bis 5 definiert ist;
b. Bereiten einer kolloidalen Suspension von Magnesiumoxid in einem organischen Lösungsmittel;
c. die erhaltene Suspension mit der dichten gemischtleitenden Membran in Kontakt bringen der erhaltenen Suspension mit der dichten gemischtleitenden Membran; und
d. Kalzinieren der erhaltenen Membran.

12. Verfahren zur Herstellung der Sauerstoff leitenden Membranen, wie sie in den Patentansprüchen 1 bis 6 und 9 bis 10 definiert sind, folgende Schritte umfassend:
a. Bereitstellen einer dichten gemischtleitenden Membran, wie sie in den Patentansprüchen 1 und 3 bis 5 definiert ist;
b. Aufbringen der Partikel aus Edelmetallen oder deren Legierungen durch Laserbedampfen.

13. Sauerstoffleitende Membran, die nach dem Verfahren nach irgendeinem der Patentansprüche 11 und 12 hergestellt worden ist.

14. Membranreaktor umfassend einen Oxidationsbereich und einen Reduktionsbereich, die durch eine Sauerstoff leitende Membran voneinander getrennt sind, wie sie in irgendeinem der Patentansprüche 1 bis 10 oder 13 definiert ist.

15. Membranreaktor nach Patentanspruch 14, in dem der Oxidationsbereich mit der Oberfläche der Membran, die mit verteilten Partikeln auf der Basis von Magnesiumoxid oder Edelmetallen beschichtet ist, in Kontakt steht.

16. Verfahren zur Oxidation eines reaktionsfähigen Gases, umfassend:
i) Verwendung eines Membranreaktors nach den Patentansprüchen 14 oder 15,
ii) Einleitung des reaktionsfähigen Gases in den Oxidationsbereich;
iii) Einleitung des sauerstoffhaltigen Gases in den Reduktionsbereich
iv) Erhitzung der die genannten Oxidations- und Reduktionsbereiche trennenden Membran auf eine Arbeitstemperatur.

17. Verfahren nach Patentanspruch 16, in dem das reaktionsfähige Gas ein leichter Kohlenwasserstoff ist, der zu Alken oxidiert wird.

18. Verfahren nach Patentanspruch 17, in dem der leichte Kohlenwasserstoff Ethan ist, das zu Ethylen oxidiert wird.

19. Verwendung eines Membranreaktors nach den Patentansprüchen 14 oder 15 zur Abscheidung von Sauerstoff aus einem sauerstoffhaltigen Gasgemisch.

## Claims

1. Oxygen-conducting membrane comprising a dense, mixed-conducting multi-metallic oxide membrane, one of the surfaces of which is coated with dispersed particles based on magnesium oxide or noble metals, wherein the dense mixed-conducting membrane comprises a multi-metallic oxide of the formula:
Saₓ Sr₁₋ₓ Co_{1-y} Fe_{y} O_{3-z}
where
0 ≤ x ≤ 1 ;
0 ≤ y ≤ 1;
z is a number which renders the charge of the compound neutral and which defines the oxygen deficiency.

2. Membrane according to claim 1, wherein the particles based on magnesium oxide and/or noble metals have a diameter comprised between 5 and 3000 nm.

3. Membrane according to claim 1 or 2, wherein the dense mixed-conducting multi-metallic oxide membrane has a perovskite structure.

4. Membrane according to claim 1, wherein the multi-metallic oxide comprises Ba_{0.5}Sr_{0.5}Co_{0.8}Fe_{0.2}O_{3-z}.

5. Membrane according to any one of the preceding claims, wherein the dense mixed-conducting multi-metallic oxide membrane has a thickness comprised between 0.5 and 10 mm.

6. Membrane according to any one of the preceding claims, wherein the particles based on magnesium oxide or noble metals represent 0.01 to 0.1% by weight of the dense membrane.

7. Membrane according to any one of the preceding claims, wherein the particles are based on magnesium oxide.

8. Membrane according to claim 7, wherein the particles based on magnesium oxide are doped using vanadium.

9. Membrane according to any one of claims 1 to 6, wherein the particles are particles of noble metals or alloys thereof.

10. Membrane according to claim 9, wherein the noble metals are selected from Pd, Pt, Rh, Ag, Au, Ru and Ir.

11. Method for preparing oxygen-conducting membranes such as that defined in claims 1 to 8, comprising steps consisting of:
a. providing a dense mixed conducting membrane such as that defined in claims 1 and 3 to 5;
b. preparing a colloidal suspension based on magnesium oxide in an organic solvent;
c. placing the suspension obtained in contact with the dense mixed-conducting membrane; and
d. calcining the membrane obtained.

12. Method for preparing oxygen-conducting membranes such as defined in claims 1 to 6 and 9 to 10, comprising steps consisting of:
a. providing a dense multi-metallic oxide membrane such as that defined in claims 1 and 3 to 5;
b. depositing the particles of noble metals or alloys thereof by means of laser vaporisation.

13. Oxygen-conducting membrane capable of being obtained by the method according to claims 11 and 12.

14. Membrane reactor comprising an oxidation zone and a reduction zone separated by an oxygen-conducting membrane such as that defined in any one of claims 1 to 10 or 13.

15. Membrane reactor according to claim 14, wherein the oxidation zone is in contact with the surface of the membrane coated with dispersed particles based on magnesium oxide or noble metals.

16. Method for oxidising a reactant gas comprising:
i) utilisation of a membrane reactor according to claim 14 or 15;
ii) introduction of the reactant gas into the oxidation zone;
iii) introduction of the gas containing oxygen into the reduction zone;
iv) heating of the membrane, separating said oxidation and reduction zones to an operating temperature.

17. Method according to claim 16, wherein the reactant gas is a light hydrocarbon which is oxidised to alkene.

18. Method according to claim 17, wherein the light hydrocarbon is ethane which is oxidised to ethylene.

19. Use of a membrane reactor according to claim 14 or 15 to recover oxygen from a gas mixture containing oxygen.
